# EUROPÄISCHE PATENTSCHRIFT

(11) **EP 1 322 911 B1**
(45) Veröffentlichungstag und Bekanntmachung des Hinweises auf die Patenterteilung: **13.12.2006**
(21) Anmeldenummer: 01986759.7
(22) Anmeldetag: 05.10.2001
(51) Int. Cl.: G01C 11/02, G03B 15/00, A61B 5/107

(54) **ANORDNUNG UND VERFAHREN ZUR ERSTELLUNG VON PHOTOGRAMMETRISCHEN BILDAUFNAHMEN**
ARRANGEMENT AND METHOD FOR PRODUCING PHOTOGRAMMETRIC IMAGE RECORDS
DISPOSITIF ET PROCEDE POUR REALISER DES CLICHES PHOTOGRAMMETRIQUES

(30) Priorität: 07.10.2000 DE 10049926
(43) Veröffentlichungstag der Anmeldung: 02.07.2003
(73) Patentinhaber: corpus.e AG, 70178 Stuttgart (DE)
(72) Erfinder: Massen, Robert, Prof. Dr., 78337 Öhningen-Wangen (DE)
(74) Vertreter: Degwert, Hartmut
(86) Internationale Anmeldenummer: PCT/EP2001/011533
(87) Internationale Veröffentlichungsnummer: WO 2002/031440

(56) Entgegenhaltungen:
- EP-B- 0 760 622
- DE-A- 4 433 197
- US-A- 4 431 290
- US-A- 4 819 660

## Beschreibung

Die Erfindung betrifft eine Anordnung und ein Verfahren zur Erstellung von Bildaufnahmen, aus denen photogrammetrisch die Raumform eines Objekts ermittelt werden kann.

Zahlreiche Produkte müssen an die individuelle Form des Objekts, z.B. eines Körperteils, angepasst werden, für das sie bestimmt sind. So müssen viele im Kontakt zum menschlichen Körper stehende Produkte wie Schuhwerk, Oberhemden, Hosen, Handschuhe, orthopädische Artikel wie Kompressionsstrümpfe und Bandagen, Sportstützen, Brillengestelle usw. passen, d.h. ihre dreidimensionale Geometrie muss der dreidimensionalen Raumform des entsprechenden Körperteils entsprechen. Diese Passbedingungen gelten nicht nur für Produkte, welche an den menschlichen Körper angepasst werden müssen, sondern im erweiterten Sinne auch für Produkte oder Komponenten, welche an technische Körper angepasst werden müssen. Als Beispiel sei die Anpassung eines Kunststoffgehäuses an den mechanischen Unterbau eines Haushaltgerätes genannt.

Die Erfassung der Raumform, d.h. der dreidimensionalen Geometrie von Körpern, geschieht heute z.B. mit sogenannten 3D-Scannern, welche in der Regel nach Verfahren der Streifenprojektion, der Lasertriangulation, der Stereovermessung, der Photogrammetrie und den Verfahren der punktweisen Abtastung der Entfernung der Oberfläche des zu erfassenden Körpers mit einem Abstandsmessgerät arbeiten. Dienen diese Geräte der Vermessung des menschlichen Körpers, so werden sie oft auch "Body-Scanner" genannt, für eher technische Anwendungen spricht man von "3D-Digitalisierern". Bei allen diesen Verfahren kommen relativ komplexe und damit auch teure elektrooptische Systeme zum Einsatz. Zur Zeit verwendete Body-Scanner kosten beispielsweise zwischen 100.000 .- und 250.000.- Euro.

In dem europäischen Patent EP 0 760 622 "Verfahren und Anordnung zur dreidimensionalen digitalisierten Erfassung der Raumform von Körpern und Körperteilen" beschreibt der Erfinder R. Massen eine extrem kostengünstige Art der Erfassung der Raumform von Körperteilen, welche auf der Mehrfach-Kamera- oder der Ein-Kamera-Photogrammetrie beruht. Hierbei wird das zu digitalisierende Körperteil mit einem eng anliegenden elastischen Überzug versehen, welcher mit photogrammetrisch auswertbaren Zielmarken versehen ist. Das Körperteil wird aus verschiedenen Raumpositionen, welche nicht unbedingt bekannt oder raumfest zu sein brauchen, aufgenommen und aus den sich überlappenden Einzelbildern werden mit Hilfe photogrammetrischer Rekonstruktionsverfahren die Raumkoordinaten der Zielmarken und daraus die dreidimensionale Form des Körpers bestimmt.

In der deutschen Patentanmeldung DPA 100 25 922.7 beschreibt der Erfinder R. Massen verschiedene Verfahren, wie durch eine Codierung des Hintergrundes zwischen punktförmigen Zielmarken in Form von Flächenmarken eine automatische Registrierung, d.h. Zuordnung der punktförmigen Zielmarken in Bildpaaren, einfacher durchgeführt werden kann. So wird z.B. beschrieben, wie durch eine flächenhafte farbige Markierung von Hintergrundregionen der punktförmigen Zielmarken durch eine einfache Farbklassifikation korrespondierende Regionen in zwei Bildern mit den Verfahren der digitalen Farbbildverarbeitung einfach bestimmt werden können.

Damit eine photogrammetrische Rekonstruktion eines Objekts möglich ist, ist es erforderlich, dass sich Bildaufnahmepaare ausreichend überlappen, d.h. dass möglichst viele gleiche Zielmarken in zwei korrespondierenden Bildaufnahmen enthalten sind, die einen zumindest teilweise überlappenden Bildbereich aufweisen.

Die Berechnung der 3D-Koordinaten der Zielmarken läßt sich umso leichter und genauer durchführen, je besser die Kamerapositionen, die während der Erstellung von Aufnahmen eines Bildpaars mit überlappenden Bildbereichen von der Kamera eingenommen wurden, zueinander ausgerichtet sind. Da in der Regel die inneren Parameter der Kamera wie Brennweite, Anzahl und Grösse der Bildpunkte (im Fall einer digitalen Kamera), Lage der optischen Achse relativ zur Bildebene usw. durch eine vorherige Kalibrierung bestimmt wurden und für alle Aufnahmen konstant sein müssen, muss die Brennweite konstant gehalten werden. Dies bedeutet, dass keine Zoom-Funktionen benutzt werden können und dass auch Autofocus-Einrichtungen ausgeschaltet werden müssen. Scharfe Bilder sind damit nur zu erhalten, wenn der Abstand der Kamera zum abzutastenden Körperteil bei allen Raumpositionen in etwa konstant ist.

Für einen Bediener, welcher die Kamera während der Erstellung der photogrammetrisch auszuwertenden Bildaufnahmen freihändig um den zu erfassenden Körperteil herum führt, ist diese Bedingung nur sehr schwer einzuhalten, wodurch eine einfache und auch für Laien mögliche Erstellung der Bildaufnahmen wesentlich erschwert wird.

Für die automatische photogrammetrische Auswertung der Bildaufnahmen des durch z.B. einen eng anliegenden Überzug markierten Körperteil sind die in der DPA 100 25 922.7 beschriebenen, mehrere punktförmige Zielmarken flächenhaft zusammenfassenden Hintergrundmarkierungen bereits sehr hilfreich. Es werden aber immer noch hohe Anforderungen an die Bildverarbeitungsverfahren gestellt, wenn die korrespondierenden Zielmarken aus Bildpaaren gefunden werden müssen, bei denen über die Orientierung der Kamera im Raum während der Erstellung der Aufnahmen nichts bekannt ist. Es bedarf dann aufwendiger und damit auch fehlerträchtiger Suchverfahren, um zu vermeiden, dass Zielmarken in den jeweilig analysierten zwei Bildaufnahmen einander zugeordnet werden, welche sich in Wirklichkeit nicht entsprechen.

Aufgabe der vorliegenden Erfindung ist es daher, eine Anordnung und ein Verfahren zur Erstellung von Bildaufnahmen zu schaffen, aus denen photogrammetrisch die Raumform eines Objekts ermittelt werden kann, die eine einfache und auch für Laien oder unerfahrene Benutzer sehr leichte Erstellung von sich im Bildbereich überlappenden Bildaufnahmen ermöglichen, die sich für eine genaue, einfache und robuste automatische photogrammetrische Auswertung eignen.

Die Aufgabe der Erfindung wird durch Anordnung zur Erstellung von Bildaufnahmen, aus denen photogrammetrisch die Raumform eines Objekts ermittelt werden kann, mit mindestens einem Bildaufnahmesystem und mehreren auf der Oberfläche des Objekts angebrachten photogrammetrisch auswertbaren Zielmarken, gelöst, die dadurch gekennzeichnet ist, daß die Anordnung darüber hinaus ein Mittel zur Erzeugung von projizierten Lichtmarkierungen, das an dem Bildaufnahmesystem oder den Bildaufnahmesystemen angebracht ist, und auf der Oberfläche des Objekts Ausrichtmarken aufweist, die sich jeweils den einzelnen für die photogrammetrische Erfassung zu erstellenden Bildaufnahmen zuordnen lassen, wobei das Mittel zur Erzeugung von projizierten Lichtmarkierungen und die den Bildaufnahmen jeweils zugeordneten Ausrichtmarken so zueinander positioniert sind, daß dann, wenn das Bildaufnahmesystem oder die Bildaufnahmesysteme vor jeder Bildaufnahme in eine solche Lage zu dem Objekt gebracht wird bzw. werden, daß von dem Mittel zur Erzeugung von projizierten Lichtmarkierungen erzeugte Lichtmarkierungen in eine vorherbestimmte Lage zu den für die jeweilige Bildaufnahme bestimmten Ausrichtmarken auf die Oberfläche des Objekts projiziert werden, die Bildaufnahmen in einer Weise erstellt werden, die eine photogrammetrische Auswertung der Bildaufnahmen anhand der Zielmarken und daraus die Bestimmung der Raumform des Objekts erlaubt.

Ferner wird die erfindungsgemäße Aufgabe durch ein Verfahren zur Erstellung von Bildaufnahmen, aus denen photogrammetrisch die Raumform eines Objekts ermittelt werden kann, gelöst, bei dem auf der Oberfläche des Objekts photogrammetrisch auswertbare Zielmarken angebracht werden und jeweils den einzelnen für die photogrammetrische Erfassung zu erstellenden Bildaufnahmen zugeordnete Ausrichtmarken vorhanden sind, mindestens ein Bildaufnahmesystem bereitgestellt wird, an dem ein Mittel zur Erzeugung von projizierten Lichtmarkierungen angebracht ist, das Bildaufnahmesystem oder die Bildaufnahmesysteme vor jeder Aufnahme in eine solche Lage zu dem Objekt gebracht wird bzw. werden, daß von dem Mittel zur Erzeugung von projizierten Lichtmarkierungen erzeugte Lichtmarkierungen in eine vorherbestimmte Lage zu den für die jeweilige Aufnahme vorgesehenen Ausrichtmarken auf die Oberfläche des Objekts projiziert werden, und die Aufnahmen mit dem Bildaufnahmesystem oder den Bildaufnahmesystemen erstellt werden.

Durch die Erfindung lassen sich auf einfache Weise Bildaufnahmen erzielen, die sich in sehr genauer Weise photogrammetrisch auswerten lassen. Das wird erfindungsgemäß dadurch erreicht, dass an dem zur photogrammetrischen Erfassung eines Objekts vorgesehenen Bildaufnahmesystem optische Hilfen angebracht sind, die Lichtmarkierungen auf dem Objekt erzeugen können, die von einem Benutzer vor der Erstellung einer Aufnahme zu Ausrichtmarken ausgerichtet werden, die auf dem Objekt angebracht sind, so daß Ausrichtung und Abstand zwischen Objekt und Bildaufnahmesystem bei den verschiedenen Aufnahmen in gewünschter Weise eingehalten werden können. Dadurch können sehr einfache und preiswerte Bildaufnahmesystemen wie sucherlose Web-Kameras für die Erstellung der Aufnahmen verwendet werden.

Vorteilhafte Weiterbildungen der Erfindung sind in den abhängigen Patentansprüchen gekennzeichnet.

Weitere Merkmale und Vorteile der Erfindung ergeben sich aus der folgenden Beschreibung eines Ausführungsbeispiels anhand der Zeichnungen. In den Zeichnungen zeigen:
Fig. 1 als Beispiel für ein photogrammetrisch zu erfassendes Objekt ein venenkrankes Bein, das sich mit Hilfe der erfindungsgemäßen Anordnung aufnehmen läßt;
Fig. 2 das mit Zielmarken und gemäß der Erfindung verwendeten Ausrichtmarken markierte Bein aus Fig. 1;
Fig. 3 eine Ausführungsform einer erfindungsgemäßen Anordnung zur Erstellung von Bildaufnahmen, aus denen photogrammetrisch die Raumform eines Objekts ermittelt werden kann, in einer Ansicht von der Seite;
Fig. 4 eine weitere Ausführungsform einer erfindungsgemäßen Anordnung zur Erstellung von Bildaufnahmen, aus denen photogrammetrisch die Raumform eines Objekts ermittelt werden kann, in einer Ansicht von der Seite.

Die erfindungsgemäße Anordnung und das erfindungsgemäße Verfahren seien beispielhaft an einem Anwendungsfall aus dem Bereich der Orthopädie, nämlich der 3D-Digitalisierung von Beinen zur automatischen Anpassung von Kompressionsstrümpfen beschrieben. Viele weitere Anwendungen sind natürlich denkbar und das beschriebene Beispiel ist daher nicht in einschränkendem Sinne aufzufassen.

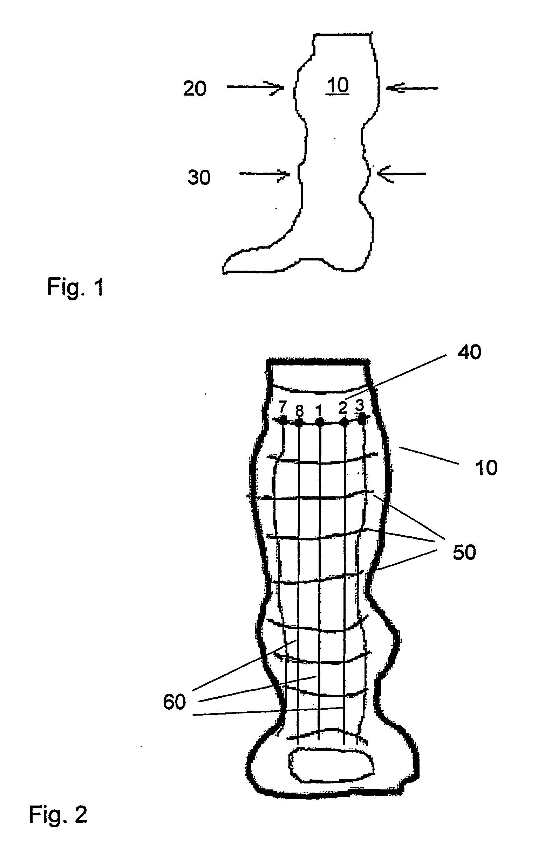

In Fig. 1 ist ein venenkrankes Bein 10 schematisch dargestellt, dessen Raumform vom Fuß aufwärts bis etwa zum Schritt photogrammetrisch erfasst werden soll, damit ein an das Bein angepasster Kompressionstrumpf so gefertigt werden kann, dass an den Stellen krankhafter Schwellungen 20 und 30 ein stärkerer Druck auf das Gewebe ausgeübt werden kann als an den gesunden Stellen.

In Fig. 2 ist das Bein 10 gemäß dem eingangs genannten europäischen Patent EP 0 760 622 mit einem markierten, eng anliegenden Überzug 40 bekleidet, wobei die photogrammetrisch auswertbaren Zielmarken durch senkrechte 50 und waagrechte 60 dunkle Linien gebildet werden, die auf dem Überzug angebracht sind. Die Kreuzungspunkte der Linien 50 und 60 bilden dabei die photogrammetrisch auswertbaren punktförmigen Zielmarken. Darüber hinaus sind beispielhaft auf dem Überzug acht Marken angebracht, die mit den Nummern 1, 2 ,3, ....8 gekennzeichnet sind. Aus perspektivischen Gründen sind in der Fig. 2 von diesen weiteren acht Marken nur die mit den Nummern 1, 2, 3, 7 und 8 gekennzeichneten Marken zu erkennen, wobei die restlichen Marken auf der anderen Seite des Überzugs angebracht sind. Die Marken 1, 2,...8 dienen als Ausrichtmarken für das Bildaufnahmesystem, mit dem die photogrammetrischen Aufnahmen erstellt werden sollen. Jede der punktförmigen Ausrichtmarken entspricht dabei einer Aufnahmeposition für die rund um das Bein herum zu erstellenden Aufnahmen. Die Ausrichtmarken sind zur Unterstützung des Bedieners des Bildaufnahmesystems, im vorliegenden Beispiel einer digitalen Farbkamera, durchnumeriert, wobei die in der Fig. 2 dargestellten Nummern 1,2,..7,8 sowohl als Bezugszeichen als auch als Nummerierung und damit Angabe der Reihenfolge dienen, in der die Punkte durch das Bildaufnahmesystem angesteuert werden müssen.

Die Kamera soll im vorliegenden Beispiel während der Erstellung der photogrammetrisch auszuwertenden Bildaufnahmen von dem Bediener freihändig in acht sich überlappenden Raumpositionen um das Bein 10 herum geführt werden.

Ohne eine Hilfe ist es für den Benutzer außerordentlich schwer, eine Kamera, welche wie in der Beschreibungseinleitung beschrieben, eine feste Brennweite und damit keine Autofokus-Möglichkeiten hat, in einem konstanten Abstand zu der Oberfläche des Beins zu führen und die Kamera so auszurichten, dass die automatische Zuordnung der Bilder anhand der Zielmarken (hier der Schnittpunkte zwischen den Linien 50 und 60) einfach möglich ist. Es ist außerdem sehr schwer, die genaue Ausrichtung und den richtigen Abstand lediglich durch den Blick durch den Sucher bzw. durch die Betrachtung des evtl. vorhandenen LCD-Sucherbildschirms zu erreichen, da der Bediener der Kamera sich auf den Patienten konzentrieren muss.

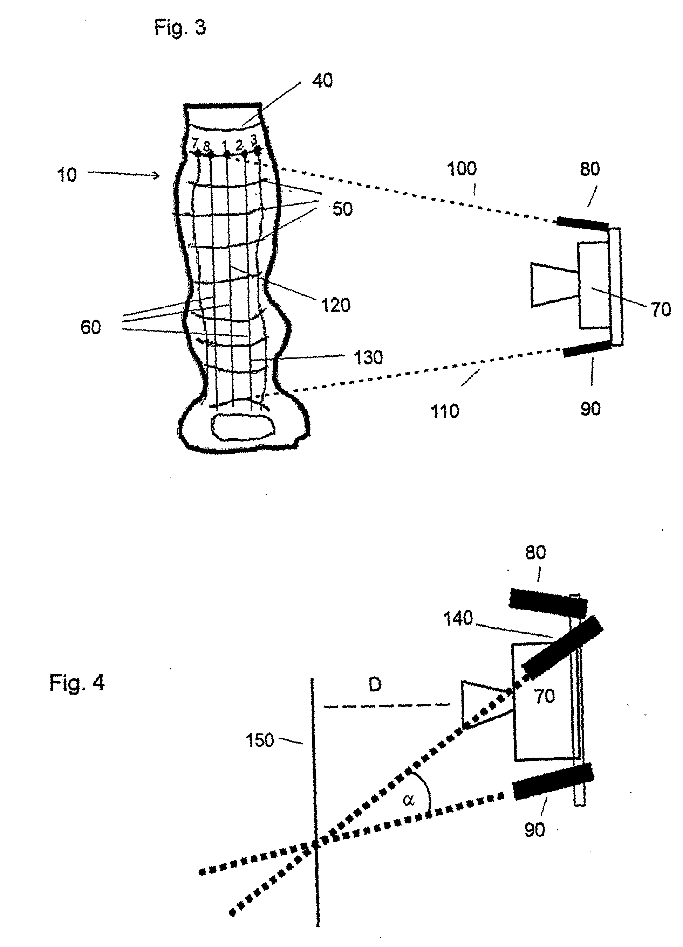

Erfindungsgemäß wird, wie in Fig. 3 gezeigt, die Kamera 70 mit zwei fest an der Kamera angebrachten Punktlichtprojektoren 80 und 90 ausgestattet, die z.B. aus zwei Laserdioden-Punktprojektoren, bestehen können. Die Punktlichtprojektoren sind, wie in der Fig. 2 zu erkennen ist, in einem solchen Winkel zueinander angeordnet, dass die von den Punktlichtprojektoren erzeugten Lichtstrahlen 100 und 110 umso stärker auseinanderlaufen, je weiter der Abstand von der Kamera ist, d.h., daß sich der Abstand zwischen den beiden Lichtstrahlen 100 und 110 mit zunehmendem Abstand der Lichtstrahlen von der Kamera zunehmend vergrößert. Die beiden Lichtstrahlen 100 und 110 markieren so beim Auftreffen auf den markierten Überzug 40 den von der Kamera 70 erfassten Bildausschnitt in einer Dimension.

Damit kann der Benutzer unmittelbar die Kamera 70 mit Hilfe der Ausrichtmarken 1, 2, 3,..., 8 so ausrichten, dass der jeweils erfasste Bildausschnitt sich in einer bekannten und damit photogrammetrisch leicht automatisch auswertbaren Lage befindet. Im vorliegenden Beispiel würde eine Anleitung zur Erstellung der ersten Bildaufnahme wie folgt aussehen:
"Richte die Kamera 70 zunächst so aus, dass der von dem oberen Punktlichtprojektor 80 erzeugte Laserpunkt die auf dem Überzug 40 aufgebrachte Ausrichtmarke 1 trifft und richte die Kamera 70 zusätzlich dabei so aus, dass der von dem unteren Punktlichtprojektor 90 erzeugte Laserpunkt auf die durch die Ausrichtmarke 1 laufende senkrechte Linie 120 trifft. In dieser Ausrichtung erstelle die erste Aufnahme durch Betätigung des Auslösers".

Diese Ausrichtung der Kamera 70 ist in der Fig. 3 dargestellt.

Die Anleitung zur Erstellung der zweiten Bildaufnahme würde dann wie folgt aussehen:
"Richte die Kamera 70 zunächst so aus, dass der von dem oberen Punktlichtprojektor 80 erzeugte Laserpunkt die auf dem Überzug aufgebrachte Ausrichtmarke 2 trifft und richte die Kamera 70 zusätzlich dabei so aus, dass der von dem unteren Punktlichtprojektor 90 erzeugte Laserpunkt auf die durch die Ausrichtmarke 1 laufende senkrechte Linie 130 trifft. In dieser Ausrichtung erstelle die zweite Aufnahme durch Betätigung des Auslösers".

Die Anleitungen zur Erstellung der Bildaufnahmen drei bis acht lauten entsprechend.

Werden in diesen, durch die optische Hilfe der Punktlichtprojektoren leicht einnehmbaren Aufnahmepositionen Nr. 1 bis 8 die Bilder aufgenommen, so entsteht ein Satz von Bildern, bei welchen die Orientierung der Kamera 70 immer gleich und parallel zu den senkrechten Markierungslinien 60 ist. Damit ist später bei der photogrammetrischen Auswertung eine automatische Zuordnung der Zielmarken aus den einzelnen, einer bekannten Systematik folgenden Bildaufnahmen (z.B. in steigender Numerierung der markierten Punkte) einfach und es können die zueinander korrespondierenden Zielmarken (hier: Kreuzungspunkte) mit Verfahren der 2D-Bildverarbeitung und Mustererkennung leicht ermittelt werden.

Dieses erfindungsgemäße Verfahren erleichtert die Ausrichtung (Drehlage und Translation) der Kamera zu dem zu digitalisierenden Körperteil während der Erstellung der Bildaufnahmen, nicht jedoch das Einhalten eines vorgegebenen Abstandes D der Kamera 70 zum Körperteil.

Erfindungsgemäß wird daher bei der in der Fig. 4 dargestellten weiteren Ausführungsform der erfindungsgemäßen Anordnung ein weiterer Punktlichtprojektor 140 so an der Kamera 70 fest angebracht, dass er in einem festen Winkel α zu einem der beiden anderen Punktlichtprojektoren, z.B. zum Projektor 90, abstrahlt. Der Winkel α ist so gewählt, dass beim Erreichen des vorgegebenen Abstandes D der vom Projektor 90 erzeugte Lichtpunkt auf den vom Projektor 140 erzeugten Lichtpunkt fällt. Dies ist für den Bediener der Kamera 70 eine sehr einfache visuelle Hilfe, um den richtigen Abstand D zwischen der Kamera 70 und dem Rand 150 des aufzunehmenden Objekts 10 einzustellen.

Ist der Abstand zwischen dem Rand 150 des aufzunehmenden Objekts und der Kamera 70 grösser oder kleiner als D, so sind zwei räumlich voneinander getrennte Lichtpunkte auf der Oberfläche des Objekts sichtbar, woran der Bediener leicht erkennen kann, daß er die Positionierung der Kamera korrigieren muß.

Die Regel zur Erstellung der ersten Aufnahme lautet bei diesem Ausführungsbeispiel demnach:
Richte die Kamera 70 zunächst so aus, dass der von dem oberen Punktlichtprojektor 80 erzeugte Laserpunkt die auf dem Überzug 40 aufgebrachte Ausrichtmarke 1 trifft und richte die Kamera 70 zusätzlich dabei so aus, dass der von dem unteren Punktlichtprojektor 90 erzeugte Laserpunkt auf die durch die Ausrichtmarke 1 laufende senkrechte Linie 120 trifft und wähle den Abstand zwischen der Kamera und dem Körperteil 10 so, daß der von dem mittleren Punktlichtprojektor 140 erzeugte Laserpunkt auf den von dem unteren Punktlichtprojektor 90 erzeugten Laserpunkt fällt. In dieser Ausrichtung erstelle die erste Aufnahme durch Betätigung des Auslösers".

Die übrigen Aufnahmen werden dann entsprechend erstellt.

Die beschriebenen Positionierungen der Kamera (durch z.B. drei projizierte Punkte) können erfindungsgemäß auch durch viele andere einfach zu projizierende Strukturen gebildet werden. Statt der in der Fig. 3 dargestellten zwei Lichtpunkte kann z.B. auch eine durchgehende Linie auf den Körper bzw. den Überzug projiziert werden. Statt durch zwei aufeinander zulaufende Lichtstrahlen von zwei Punktlichtprojektoren kann die Einstellung des Abstands auch z.B. durch die Projektion eines Kreises visualisiert werden. Dabei wird der Kreisdurchmesser beispielsweise so gewählt, dass beim korrekten Abstand der sichtbare Kreis zu einem Punkt schrumpft.

Der Erfindungsgedanke umfasst auch alle projizierbaren Strukturen, welche die Ausrichtung der Kamera zum Körperteil bzw. zu den Markierungen des den Köperteil bekleidenden Überzugs visuell ermöglichen, ohne das Sucherbild der Kamera oder das von der Kamera erzeugte elektronische Bild zur Ausrichtung betrachten zu müssen sowie alle projizierbaren Strukturen, welche es visuell ermöglichen, den korrekten Abstand zum Körperteil einzunehmen.

Die aufprojizierten Strukturen können auch mehrfarbig und darüber hinaus so gestaltet sein, daß sich durch Überlagerung der Strukturen beim korrekten Abstand zwischen Kamera und Objekt, ein sichtbarer Farbwechsel am Ort der Überlagerung ergibt, der als Indiz für die korrekte Lage der Kamera dient.

Die optische Hilfe in Form einer Projektionseinrichtung kann auch an einer Videokamera angebaut werden, um bei laufender Videokamera photogrammetrisch einfach auswertbare, einfach zueinander orientierte und im korrekten Abstand aufgenommene Bildsequenzen zu erzeugen.

Die Projektoren können bei einer weiteren Ausführungsform auch vor der Bildaufnahme ausgeschaltet werden, damit die projizierten Strukturen die photogrammetrischen Zielmarken bei der Bildaufnahme nicht stören.

Die Ausrichtmarken können bei einer weiteren Ausführungsform der Erfindung auch in einer für das Auge im Kontrast zum Hintergrund gut sichtbaren Farbe projiziert werden. Mit Verfahren der Farbbildverarbeitung können diese Farben automatisch aus den Bildaufnahmen wieder entfernt werden, so dass die Projektoren während der eigentlichen Bildaufnahme nicht ausgeschaltet werden müssen.

Bei einer weiteren Ausführungsform bleiben die Projektoren während der Bildaufnahme ebenfalls eingeschaltet und die projizierten Strukturen werden mit Verfahren der Bildverarbeitung ausgewertet, um Informationen darüber aus den Bildaufnahmen abzuleiten, ob die Aufnahmen überhaupt in korrekter Weise erstellt wurden, ob also z.B. die projizierten Strukturen auf den Ausrichtmarken lagen. So kann z.B. die Auswertung der auf den Abstand reagierenden Struktur Aussagen ergeben, ob das Bild im richtigen Abstand aufgenommen wurde.

Die projizierten Strukturen zur Überprüfung eines korrekten Abstandes oder einer korrekten Orientierung können auch bei üblichen, ausserhalb der Photogrammetrie eingesetzten Kameras zum Einsatz kommen, um dem Benutzer zu helfen, einen korrekten Abstand von einem aufzunehmenden Objekt einzuhalten oder eine korrekte Ausrichtung der Kamera zu dem Objekt zu erreichen.

Bei einer weiteren Ausführungsform können die Lichtmusterprojektoren mechanisch an einer stabilen Schiene befestigt sein und über eine Stativverschraubung am Kamerakörper dort befestigt werden, wo sich sonst eine Befestigung für ein Stativ befindet.

Bei einer weiteren Ausführungsform der Erfindung brauchen die Ausrichtmarken nicht extra auf dem Objekt oder einem Überzug angebracht sein, sondern sie können aus natürlichen markanten Punkten des Objekts bestehen (z.B. aus Adern, die an einem Bein sichtbar sind).

Die Ziel- und Ausrichtmarken müssen auch nicht unbedingt auf einem Überzug angebracht sein, sie können auch z.B. durch ein weiteres Projektionssystem auf das Objekt projiziert werden können oder direkt auf das Objekt (z.B. mit Farbe) aufgebracht werden.

Die Mittel zur Erzeugung von projizierten Lichtmarkierungen müssen nicht notwendigerweise Laser sein, sondern können auch aus anderen lichterzeugenden Elementen bestehen und z.B. auch LEDs aufweisen.

Das zu digitalisierende Objekt muß nicht unbedingt ein Körperteil des Menschen sein, sondern kann ein beliebiges Objekt sein, dessen Raumform bestimmt werden soll.

Die Erfindung schafft im übrigen auch eine Anordnung zur photogrammetrischen Erfassung der Raumform eines Objekts mit einer erfindungsgemäßen Anordnung zur Erstellung von Bildaufnahmen sowie einem herkömmlichen System zur Vermessung und Auswertung der Bildaufnahmen sowie zur Ermittlung der Raumform des Objekts.

## Patentansprüche

1. Anordnung zur Erstellung von Bildaufnahmen, aus denen photogrammetrisch die Raumform eines Objekts ermittelt werden kann, mit mindestens einem Bildaufnahmesystem (70) und mehreren auf der Oberfläche des Objekts (10) angebrachten photogrammetrisch auswertbaren Zielmarken (50,60), **dadurch gekennzeichnet, daß** die Anordnung darüber hinaus ein Mittel (80, 90, 140) zur Erzeugung von projizierten Lichtmarkierungen, das an dem Bildaufnahmesystem (70) oder den Bildaufnahmesystemen angebracht ist, und auf der Oberfläche des Objekts (10) Ausrichtmarken (1,2,3,...,8) aufweist, die sich jeweils den einzelnen für die photogrammetrische Erfassung zu erstellenden Bildaufnahmen zuordnen lassen, wobei das Mittel (80, 90, 140) zur Erzeugung von projizierten Lichtmarkierungen und die den Bildaufnahmen jeweils zugeordneten Ausrichtmarken (1,2,3,...,8) so zueinander positioniert sind, daß dann, wenn das Bildaufnahmesystem (70) oder die Bildaufnahmesysteme vor jeder Bildaufnahme in eine solche Lage zu dem Objekt (10) gebracht wird bzw. werden, daß von dem Mittel zur Erzeugung von projizierten Lichtmarkierungen erzeugte Lichtmarkierungen in eine vorherbestimmte Lage zu den für die jeweilige Bildaufnahme bestimmten Ausrichtmarken (1, 2, 3,...,8) auf die Oberfläche des Objekts (10) projiziert werden, die Bildaufnahmen in einer Weise erstellt werden, die eine photogrammetrische Auswertung der Bildaufnahmen anhand der Zielmarken (50, 60) und daraus die Bestimmung der Raumform des Objekts (10) erlaubt.

2. Anordnung nach Anspruch 1, bei der das Mittel zur Erzeugung von projizierten Lichtmarkierungen und die den Bildaufnahmen jeweils zugeordneten Ausrichtmarken so zueinander positioniert sind, daß dann, wenn das Bildaufnahmesystem oder die Bildaufnahmesysteme vor jeder Bildaufnahme in eine solche Lage zu dem Objekt gebracht wird bzw. werden, daß von dem Mittel zur Erzeugung von projizierten Lichtmarkierungen erzeugte Lichtmarkierungen in eine vorherbestimmte Lage zu den für die jeweilige Bildaufnahme bestimmten Ausrichtmarken auf die Oberfläche des Objekts projiziert werden, die Ausrichtung zwischen dem Bildaufnahmesystem oder den Bildaufnahmesystemen und dem Objekt bei jeder Bildaufnahme konstant bleibt.

3. Anordnung nach Anspruch 1, bei der das Mittel zur Erzeugung von projizierten Lichtmarkierungen und die den Bildaufnahmen jeweils zugeordneten Ausrichtmarken so zueinander positioniert sind, daß dann, wenn das Bildaufnahmesystem oder die Bildaufnahmesysteme vor jeder Bildaufnahme in eine solche Lage zu dem Objekt gebracht wird bzw. werden, daß von dem Mittel zur Erzeugung von projizierten Lichtmarkierungen erzeugte Lichtmarkierungen in eine vorherbestimmte Lage zu den für die jeweilige Bildaufnahme bestimmten Ausrichtmarken auf die Oberfläche des Objekts projiziert werden, der Abstand zwischen dem Bildaufnahmesystem oder den Bildaufnahmesystemen und dem Objekt bei jeder Bildaufnahme konstant bleibt.

4. Anordnung nach Anspruch 2 und 3, bei der das Mittel zur Erzeugung von projizierten Lichtmarkierungen und die den Bildaufnahmen jeweils zugeordneten Ausrichtmarken so zueinander positioniert sind, daß dann, wenn das Bildaufnahmesystem oder die Bildaufnahmesysteme vor jeder Bildaufnahme in eine solche Lage zu dem Objekt gebracht wird bzw. werden, daß von dem Mittel zur Erzeugung von projizierten Lichtmarkierungen erzeugte Lichtmarkierungen in eine vorherbestimmte Lage zu den für die jeweilige Bildaufnahme bestimmten Ausrichtmarken auf die Oberfläche des Objekts projiziert werden, der Abstand und die Ausrichtung zwischen dem Bildaufnahmesystem oder den Bildaufnahmesystemen und dem Objekt bei jeder Bildaufnahme konstant bleiben.

5. Anordnung nach einem der vorhergehenden Ansprüche, bei der die projizierten Lichtmarkierungen punktförmig sind.

6. Anordnung nach einem der Ansprüche 1 bis 4, bei der die projizierten Lichtmarkierungen linienförmig sind.

7. Anordnung nach Anspruch 2 und 5, bei der die Mittel zur Erzeugung von projizierten Lichtmarkierungen in einem Winkel zueinander angeordnet sind und die Ausrichtmarken aus einem Punkt und einer mit diesem Punkt verbundenen Gerade bestehen.

8. Anordnung nach Anspruch 2 und 5, bei der die Mittel zur Erzeugung von projizierten Lichtmarkierungen aus zwei Lichtprojektoren (80, 90) bestehen, die in einem solchen Winkel zueinander angeordnet sind, daß die von Ihnen erzeugten Lichtstrahlen auseinanderlaufen und die Ausrichtmarken für eine Bildaufnahme jeweils aus einem Punkt und einer mit diesem Punkt verbundenen Gerade bestehen.

9. Anordnung nach Anspruch 3 und 5, bei der die Mittel zur Erzeugung von projizierten Lichtmarkierungen aus zwei Lichtprojektoren (90, 140) bestehen, die in einem solchen Winkel zueinander angeordnet sind, daß die von Ihnen erzeugten Lichtstrahlen aufeinander zulaufen und die Ausrichtmarken für eine Bildaufnahme jeweils aus einem Punkt bestehen.

10. Anordnung nach Anspruch 4 und 5, bei der die Mittel zur Erzeugung von projizierten Lichtmarkierungen aus drei Lichtprojektoren bestehen, von denen zwei (80, 90) in einem solchen Winkel angeordnet sind, daß die von Ihnen erzeugten Lichtstrahlen auseinanderlaufen, wobei der dritte Lichtprojektor (140) in einem solchen Winkel zu wenigstens einem der beiden Lichtprojektoren steht, daß die von dem dritten Lichtprojektor und dem einen der beiden Lichtprojektoren erzeugten Lichtstrahlen aufeinander zulaufen und die Ausrichtmarken für eine Bildaufnahme jeweils aus einem Punkt und einer mit diesem Punkt verbundenen Gerade bestehen.

11. Anordnung nach einem der vorhergehenden Ansprüche, bei der die Ausrichtmarken entsprechend der Reihenfolge der zu erstellenden Bildaufnahmen durchnummeriert sind.

12. Anordnung nach einem der vorhergehenden Ansprüche, bei der die Ausrichtmarken aus markanten Punkten des Objekts bestehen.

13. Anordnung nach einem der Ansprüche 1 bis 11, die darüber hinaus einen elastischen Überzug aufweist, auf dem die Zielmarken und die Ausrichtmarken angebracht sind und der so ausgebildet ist, daß er über das Objekt gezogen werden kann und sich dabei an die Form des Objekts anpaßt.

14. Anordnung nach einem der Ansprüche 1 bis 11, die darüber hinaus ein Projektionssystem umfaßt, das so ausgebildet ist, daß damit die Zielmarken und die Ausrichtmarken auf das Objekt projiziert werden können.

15. Anordnung nach einem der vorhergehenden Ansprüche, bei der das Bildaufnahmesystem aus einer Digitalkamera ohne Sucher besteht, die während der Aufnahmen freihändig um das Objekt geschwenkt werden kann.

16. Anordnung nach einem der vorhergehenden Ansprüche, bei der die Mittel zur Erzeugung von projizierten Lichtmarkierungen Laser sind.

17. Anordnung nach einem der Ansprüche 1 bis 15, bei der die Mittel zur Erzeugung von projizierten Lichtmarkierungen LEDs sind.

18. Anordnung nach einem der vorhergehenden Ansprüche, bei der das Objekt ein Körperteil eines Menschen ist.

19. Anordnung zur photogrammetrischen Erfassung der Raumform eines Objekts mit einer Anordnung zur Erstellung von Bildaufnahmen nach einem der vorhergehenden Ansprüche und einem System zur Vermessung und Auswertung der Bildaufnahmen sowie zur Ermittlung der Raumform des Objekts.

20. Verfahren zur Erstellung von Bildaufnahmen, aus denen photogrammetrisch die Raumform eines Objekts ermittelt werden kann, bei dem auf der Oberfläche des Objekts photogrammetrisch auswertbare Zielmarken angebracht werden und jeweils den einzelnen für die photogrammetrische Erfassung zu erstellenden Bildaufnahmen zugeordnete Ausrichtmarken vorhanden sind, mindestens ein Bildaufnahmesystem bereitgestellt wird, an dem ein Mittel zur Erzeugung von projizierten Lichtmarkierungen angebracht ist, das Bildaufnahmesystem oder die Bildaufnahmesysteme vor jeder Aufnahme in eine solche Lage zu dem Objekt gebracht wird bzw. werden, daß von dem Mittel zur Erzeugung von projizierten Lichtmarkierungen erzeugte Lichtmarkierungen in eine vorherbestimmte Lage zu den für die jeweilige Aufnahme vorgesehenen Ausrichtmarken auf die Oberfläche des Objekts projiziert werden, und die Aufnahmen mit dem Bildaufnahmesystem oder den Bildaufnahmesystemen erstellt werden.

21. Verfahren nach Anspruch 20, bei dem das Mittel zur Erzeugung von projizierten Lichtmarkierungen während der Erstellung der Bildaufnahmen ausgeschaltet wird, damit die Lichtmarkierungen nicht auf den Bildaufnahmen erscheinen.

22. Verfahren nach einem der Ansprüche 20 oder 21, bei dem auch die Zielmarken auf dem Objekt angebracht werden.

23. Verfahren nach Anspruch 22, bei dem das Anbringen der Ziel- und Ausrichtmarken in der Weise erfolgt, daß ein elastischer Überzug, auf dem die Ziel- und Ausrichtmarken aufgebracht sind, über das Objekt gezogen wird.

24. Verfahren nach Anspruch 22, bei dem das Anbringen der Ziel- und Ausrichtmarken in der Weise erfolgt, daß diese auf die Oberfläche des Objekts projiziert werden.

25. Verfahren nach einem der Ansprüche 20 bis 24, bei das Bildaufnahmesystem aus einer sucherlosen Digitalkamera besteht, die freihändig um das Objekt herumgeführt wird.

26. Verfahren zur photogrammetrischen Erfassung der Raumform eines Objekts, bei dem mehrere Bildaufnahmen nach einem der Ansprüche 20 bis 25 erstellt werden, eine Bildverarbeitung durchgeführt wird, bei der die sich in den Bildaufnahmen jeweils entsprechenden Zielmarken einander zugeordnet werden und dann anhand der Zielmarkenzuordnung die Raumform des Objekts mit einem photogrammetrischen Auswerteverfahren ermittelt wird.

## Claims

1. An arrangement for obtaining image recordings from which the 3D shape of an object can be determined by photogrammetry, comprising at least one image recording system (70) and a plurality of target markers (50, 60) applied on the surface of the object (10) and adapted to be evaluated photogrammetrically, **characterized in that** the arrangement further includes a means (80, 90, 140) for generating projected light markings which is mounted to the image recording system or systems (70) and alignment markers (1, 2, 3, ..., 8) on the surface of the object (10) each of which can be associated with an individual one of the image recordings to be obtained for the photogrammetric sensing, the means (80, 90, 140) for generating projected light markings and the alignment markers (1, 2, 3, ..., 8) respectively associated with the image recordings being positioned in relation to each other such that when the image recording system or systems (70), prior to recording each image, is/are positioned in relation to the object (10) such that light markings generated by the means for generating projected light markings are projected onto the surface of the object (10) in a predetermined position in relation to the alignment markers (1, 2, 3, ..., 8) intended for the respective image recording, the image recordings are obtained in a manner so as to allow a photogrammetric evaluation of the image recordings using the target markers (50, 60) and, from this, the determination of the 3D shape of the object (10).

2. The arrangement according to claim 1, wherein the means for generating projected light markings and the alignment markers respectively associated with the image recordings are positioned in relation to each other such that when the image recording system or systems, prior to recording each image, is/are positioned in relation to the object such that light markings generated by the means for generating projected light markings are projected onto the surface of the object in a predetermined position in relation to the alignment markers intended for the respective image recording, the alignment between the image recording system or systems and the object remains constant while each image is recorded.

3. The arrangement according to claim 1, wherein the means for generating projected light markings and the alignment markers respectively associated with the image recordings are positioned in relation to each other such that when the image recording system or systems, prior to recording each image, is/are positioned in relation to the object such that light markings generated by the means for generating projected light markings are projected onto the surface of the object in a predetermined position in relation to the alignment markers intended for the respective image recording, the distance between the image recording system or systems and the object remains constant while each image is recorded.

4. The arrangement according to claims 2 and 3, wherein the means for generating projected light markings and the alignment markers respectively associated with the image recordings are positioned in relation to each other such that when the image recording system or systems, prior to recording each image, is/are positioned in relation to the object such that light markings generated by the means for generating projected light markings are projected onto the surface of the object in a predetermined position in relation to the alignment markers intended for the respective image recording, the distance and the alignment between the image recording system or systems and the object remain constant while each image is recorded.

5. The arrangement according to any of the preceding claims, wherein the projected light markings are punctiform.

6. The arrangement according to any of claims 1 to 4, wherein the projected light markings are in the shape of a line.

7. The arrangement according to claims 2 and 5, wherein the means for generating projected light markings are arranged at an angle to each other and the alignment markers consist of a point and a straight line connected with this point.

8. The arrangement according to claims 2 and 5, wherein the means for generating projected light markings consist of a pair of light projectors (80, 90) arranged at an angle to each other such that the light beams generated by them diverge and the alignment markers for one image recording each consist of a point and a straight line connected with this point.

9. The arrangement according to claims 3 and 5, wherein the means for generating projected light markings consist of a pair of light projectors (90, 140) arranged at an angle to each other such that the light beams generated by them converge and the alignment markers for one image recording each consist of a point.

10. The arrangement according to claims 4 and 5, wherein the means for generating projected light markings consist of three light projectors, two of which (80, 90) are arranged at an angle such that the light beams generated by them diverge, the third light projector (140) being at an angle to at least one of the two light projectors such that the light beams generated by the third light projector and the one of the two light projectors converge and the alignment markers for one image recording each consist of a point and a straight line connected with this point.

11. The arrangement according to any of the preceding claims, wherein the alignment markers are numbered consecutively in accordance with the order of the image recordings to be obtained.

12. The arrangement according to any of the preceding claims, wherein the alignment markers consist of distinct points of the object.

13. The arrangement according to any of claims 1 to 11, further including an elastic envelope which has the target markers and the alignment markers applied thereon and is designed so as to be pulled over the object and to adapt to the shape of the object.

14. The arrangement according to any of claims 1 to 11, further comprising a projection system which is designed to be used for projecting the target markers and the alignment markers onto the object.

15. The arrangement according to any of the preceding claims, wherein the image recording system is comprised of a digital camera without a viewfinder, which can be panned free-handed around the object while the images are recorded.

16. The arrangement according to any of the preceding claims, wherein the means for generating projected light markings are lasers.

17. The arrangement according to any of claims 1 to 15, wherein the means for generating projected light markings are LEDs.

18. The arrangement according to any of the preceding claims, wherein the object is a part of a human body.

19. An arrangement for photogrammetric sensing of the 3D shape of an object, comprising an arrangement for obtaining image recordings according to any of the preceding claims and a system for measuring and evaluating the recorded images and for determining the 3D shape of the object.

20. A method of obtaining image recordings from which the 3D shape of an object can be determined by photogrammetry, wherein target markers adapted to be evaluated photogrammetrically are applied onto the surface of the object and alignment markers are provided each of which is associated with an individual one of the image recordings to be obtained for the photogrammetric sensing, at least one image recording system is provided which has mounted thereto a means for generating projected light markings, the image recording system or systems, prior to recording each image, is/are positioned in relation to the object such that light markings generated by the means for generating projected light markings are projected onto the surface of the object in a predetermined position in relation to the alignment markers provided for the respective recording, and the recordings are obtained using the image recording system or systems.

21. The method according to claim 20, wherein the means for generating projected light markings is turned off while the image recordings are obtained so as to prevent the light markings from appearing on the recorded images.

22. The method according to either one of claims 20 or 21, wherein the target markers are also applied onto the object.

23. The method according to claim 22, wherein the application of the target and alignment markers is effected in such a way that an elastic envelope which has the target and alignment markers applied thereon is pulled over the object.

24. The method according to claim 22, wherein the application of the target and alignment markers is effected in such a way that they are projected on the surface of the object.

25. The method according to any of claims 20 to 24, wherein the image recording system is comprised of a digital camera without a viewfinder, which is guided free-handed around the object.

26. A method of sensing the 3D shape of an object by photogrammetry, wherein a plurality of image recordings are obtained in accordance with any of claims 20 to 25, an image processing is performed in which the target markers respectively corresponding in the image recordings are associated with each other and then, using the target marker association, the 3D shape of the object is determined by means of a photogrammetric evaluation process.

## Revendications

1. Agencement pour réaliser des prises de vues, à partir desquelles la forme spatiale d'un objet peut être déterminée par photogrammétrie, comportant au moins un système de prises de vues (70) et plusieurs repères de visée (50, 60) appliqués sur la surface de l'objet (10) et analysables par photogrammétrie, **caractérisé en ce que** l'agencement présente en outre des moyens (80, 90, 140) de génération de marques lumineuses projetées, qui est monté sur le ou les système(s) de prises de vues (70), et des repères d'alignement (1, 2, 3, ..., 8) sur la surface de l'objet (10) qui peuvent être chacun associés aux prises de vues individuelles à réaliser pour la détection photogrammétrique, les moyens (80, 90, 140) de génération de marques lumineuses projetées et les repères d'alignement (1, 2, 3, ..., 8) associés aux prises de vues respectives étant positionnés les uns par rapport aux autres de telle sorte que lorsque le ou les système(s) de prises de vues (70) est/sont amené(s) avant chaque prise de vue dans une position telle par rapport à l'objet (10) que des marques lumineuses, engendrées par les moyens de génération de marques lumineuses projetées, sont projetées sur la surface de l'objet (10) dans une position prédéterminée par rapport aux repères d'alignement (1, 2, 3, ..., 8) destinés pour la prise de vue respective, les prises de vues sont réalisées d'une manière à permettre une analyse photogrammétrique des prises de vues à l'aide des repères de visée (50, 60) et, à partir de celle-ci, la détermination de la forme spatiale de l'objet (10).

2. Agencement selon la revendication 1, dans lequel les moyens de génération de marques lumineuses projetées et les repères d'alignement associés à chacune des prises de vues sont positionnés de telle sorte les uns par rapport aux autres que lorsque le ou les système(s) de prises de vues est/sont amené(s) avant chaque prise de vue dans une position telle par rapport à l'objet que des marques lumineuses, engendrées par les moyens de génération de marques lumineuses projetées, sont projetées sur la surface de l'objet dans une position prédéterminée par rapport aux repères d'alignement destinés pour la prise de vue respective, l'alignement entre le ou les système(s) de prises de vues et l'objet reste constant à chaque prise de vue.

3. Agencement selon la revendication 1, dans lequel les moyens de génération de marques lumineuses projetées et les repères d'alignement associés à chacune des prises de vues sont positionnés de telle sorte les uns par rapport aux autres que lorsque le ou les système(s) de prises de vues est/sont amené(s) avant chaque prise de vue dans une position telle par rapport à l'objet que des marques lumineuses, engendrées par les moyens de génération de marques lumineuses projetées, sont projetées sur la surface de l'objet dans une position prédéterminée par rapport aux repères d'alignement destinés pour la prise de vue respective, la distance entre le ou les système(s) de prises de vues et l'objet reste constante à chaque prise de vue.

4. Agencement selon la revendication 2 et 3, dans lequel les moyens de génération de marques lumineuses projetées et les repères d'alignement associés à chacune des prises de vues sont positionnés de telle sorte les uns par rapport aux autres que lorsque le ou les système(s) de prises de vues est/sont amené(s) avant chaque prise de vue dans une position telle par rapport à l'objet que des marques lumineuses, engendrées par les moyens de génération de marques lumineuses projetées, sont projetées sur la surface de l'objet dans une position prédéterminée par rapport aux repères d'alignement destinés pour la prise de vue respective, la distance et l'alignement entre le ou les système(s) de prises de vues et l'objet restent constants à chaque prise de vue.

5. Agencement selon l'une des revendications précédentes, dans lequel les marques lumineuses projetées sont en forme de points.

6. Agencement selon l'une des revendications 1 à 4, dans lequel les marques lumineuses projetées sont en forme de lignes.

7. Agencement selon la revendication 2 et 5, dans lequel les moyens de génération de marques lumineuses projetées sont agencés sous un angle les uns par rapport aux autres et les repères d'alignement sont constitués par un point et par une droite reliée à ce point.

8. Agencement selon la revendication 2 et 5, dans lequel les moyens de génération de marques lumineuses projetées sont constitués par deux projecteurs lumineux (80, 90) qui sont agencés sous un angle tel l'un par rapport à l'autre que les rayons lumineux qu'ils engendrent divergent et les repères d'alignement pour une prise de vue sont constitués chacun par un point et par une droite reliée à ce point.

9. Agencement selon la revendication 3 et 5, dans lequel les moyens de génération de marques lumineuses projetées sont constitués par deux projecteurs lumineux (80, 90) qui sont agencés sous un angle tel l'un par rapport à l'autre que les rayons lumineux qu'ils engendrent convergent et les repères d'alignement pour une prise de vue sont constitués chacun par un point.

10. Agencement selon la revendication 4 et 5, dans lequel les moyens de génération de marques lumineuses projetées sont constitués par trois projecteurs lumineux dont deux (80, 90) qui sont agencés sous un angle tel l'un par rapport à l'autre que les rayons lumineux qu'ils engendrent divergent, le troisième projecteur lumineux (140) étant sous un angle tel par rapport à au moins l'un des deux projecteurs lumineux que les rayons lumineux engendrés par le troisième projecteur lumineux et par l'un des deux projecteurs lumineux sont convergents, et les repères d'alignement pour une prise de vue sont constitués chacun par un point et par une droite reliée à ce point.

11. Agencement selon l'une des revendications précédentes, dans lequel les repères d'alignement sont numérotés selon l'ordre des prises de vues à réaliser.

12. Agencement selon l'une des revendications précédentes, dans lequel les repères d'alignement sont constitués par des points marquants de l'objet.

13. Agencements selon l'une des revendications 1 à 11, qui présente en outre un revêtement élastique sur lequel sont appliqués des repères de visée et des repères d'alignement et qui est réalisé de manière à pouvoir être tiré au-dessus de l'objet et à s'adapter à la forme de l'objet.

14. Agencement selon l'une des revendications 1 à 11, qui comprend en outre un système de projection qui est réalisé de manière à ce qu'avec celui-ci, les repères de visée et les repères d'alignement puissent être projetés sur l'objet.

15. Agencement selon l'une des revendications précédentes, dans lequel le système de prises de vues est constitué par une caméra numérique sans viseur, qui peut être pivotée à main libre autour de l'objet pendant la prise de vue.

16. Agencement selon l'une des revendications précédentes, dans lequel les moyens de génération de marques lumineuses projetées sont des lasers.

17. Agencement selon l'une des revendications 1 à 15, dans lequel les moyens de génération de marques lumineuses projetées sont des diodes électroluminescentes.

18. Agencement selon l'une des revendications précédentes, dans lequel l'objet est une partie d'un corps humain.

19. Agencement pour la détection photogrammétrique de la forme spatiale d'un objet avec un agencement pour réaliser des prises de vues selon l'une des revendications précédentes et un système de mesure et d'analyse des prises de vues ainsi que pour déterminer la forme spatiale de l'objet.

20. Procédé de réalisation de prises de vues à partir desquelles la forme spatiale d'un objet peut être déterminée par photogrammétrie, dans lequel des repères de visée analysables par photogrammétrie sont appliqués sur la surface de l'objet, dans lequel sont en présence des repères d'alignement qui sont chacun associés aux prises de vues individuelles à réaliser pour la détection photogrammétrique, dans lequel est fourni au moins un système de prises de vues, sur lequel sont montés des moyens de génération de marques lumineuses projetées, dans lequel le ou les système(s) de prises de vue est/sont amené(s) avant chaque prise de vue dans une position telle par rapport à l'objet que des marques lumineuses engendrées par les moyens de génération de marques lumineuses projetées sont projetées sur la surface de l'objet dans une position prédéterminée par rapport aux repères d'alignement prévus pour la prise de vue respective, et dans lequel les prises de vues sont réalisées avec le ou les système(s) de prises de vues.

21. Procédé selon la revendication 20, dans lequel les moyens de génération de marques lumineuses projetées sont déconnectés pendant la réalisation de prises de vues, afin que les marques lumineuses n'apparaissent pas sur les prises de vues.

22. Procédé selon l'une des revendications 20 ou 21, dans lequel les repères de visée sont aussi appliqués sur l'objet.

23. Procédé selon la revendication 22, dans lequel l'application de repères de visée et de repères d'alignement a lieu de telle sorte qu'un revêtement élastique, sur lequel sont appliqués les repères de visée et les repères d'alignement, est tiré au-dessus de l'objet.

24. Procédé selon la revendication 22, dans lequel l'application de repères de visée et de repères d'alignement a lieu de telle sorte que ceux-ci sont projetés sur la surface de l'objet.

25. Procédé selon l'une des revendications 20 à 24, dans lequel le système de prises de vue est constituée par une caméra numérique sans viseur qui est dirigée à main libre autour de l'objet.

26. Procédé de détection photogrammétrique de la forme spatiale d'un objet, dans lequel plusieurs prises de vues sont réalisées selon l'une des revendications 20 à 25, un traitement d'image est effectué, dans lequel les repères de visée qui se correspondent dans les prises de vues sont respectivement associés les uns aux autres et ensuite, à l'aide de la correspondance entre les repères de visée, la forme spatiale de l'objet est déterminée par un procédé d'analyse photogrammétrique.
